(19)

Europäisches Patentamt
European Patent Office
Office européen des brevets

(11)  **EP 2 650 669 A1**

(12)  **EUROPEAN PATENT APPLICATION**
published in accordance with Art. 153(4) EPC

(43) Date of publication:
**16.10.2013 Bulletin 2013/42**

(21) Application number: **11847009.5**

(22) Date of filing: **07.12.2011**

(51) Int Cl.:
**G01N 15/06** [(2006.01)]          **G01N 1/10** [(2006.01)]
**G01N 21/49** [(2006.01)]          **G01N 33/44** [(2006.01)]

(86) International application number:
**PCT/JP2011/078317**

(87) International publication number:
**WO 2012/077716 (14.06.2012 Gazette 2012/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2010 JP 2010272495**

(71) Applicant: **Bridgestone Corporation Tokyo 104-8340 (JP)**

(72) Inventor: **ISHINO Yuichi Kodaira-shi Tokyo 187-0031 (JP)**

(74) Representative: **Lamb, Martin John Carstairs Marks & Clerk LLP 90 Long Acre London WC2E 9RA (GB)**

(54)  **RUBBER ANALYSIS METHOD**

(57)  Provided is a rubber analysis method in which an analysis of natural rubber and/or a diene-based synthetic rubber including an ultra-high molecular weight component which could not be analyzed in a conventional method.

The method is a rubber analysis method comprising: a dissolution process in which natural rubber and/or diene-based synthetic rubber is/are dissolved in an organic solvent; and a separation process in which a solution in which the natural rubber and/or diene-based synthetic rubber is/are dissolved is subjected to a centrifugation at a centrifugal acceleration of from 10,000 G to 1,000,000 G, to separate a soluble component and an insoluble component in the solution.

EP 2 650 669 A1

**Description**

Technical Field

The present invention relates to a rubber analysis method (hereinafter, also simply referred to as "analysis method"), and in particular, to a rubber analysis method for evaluating a higher-order structure such as the molecular weight, branches and gel fraction of an ultra-high molecular weight component, and natural rubber and/or a diene-based synthetic rubber having many branches.

Background Art

In recent years, the importance of natural rubber is ever-increasing, in view of environmental issues and its excellent physical properties. For the development of a natural rubber provided with a new function and for managing produced natural rubber, it is important to comprehend a basic structure parameter such as molecular weight. Regarding not only natural rubber but also a diene-based synthetic rubber, for managing produced synthetic rubber, it is important to comprehend a basic structure parameter such as molecular weight.
Conventionally, for the analysis of the high- order structure of natural rubber and a diene- based synthetic rubber, the following procedure has been used: Rubber is dissolved in an organic solvent such as tetrahydrofuran (THF), and then impurities such as rubber components which is insoluble in THF is filtrated with a filter, whereby molecular weight distribution is measured by gel permeation chromatography (GPC) . For example, Non- Patent Documents 1 to 3 disclose use of a field flow fractionation (hereinafter, also referred to as "FFF") device or an FFF and multi- angle light scattering (hereinafter, also referred to as "MALS") detector, for structural analysis of natural rubber or a diene- based synthetic rubber. In these Documents, pretreatment method of a sample or analysis method of analysis data is not appropriate, and the procedure has been unsatisfactory for an analysis method of the higher- order structure of natural rubber or a diene- based synthetic rubber.

Related Art Documents

Non-Patent Documents

Non-Patent Document 1: "Journal of Natural Rubber Research", 1997, Vol.12(3), p.154-165
Non-Patent Document 2: "Macromolecules", 1995, 28, p.6354-6356
Non-Patent Document 3: "Bull. Korean Chem. Soc.", 2000, Vol.21, No.1, p.69-74

Summary of the Invention

Problems to be Solved by the Invention

Conventionally, since information of the average molecular weight or the molecular weight distribution of natural rubber or a diene- based synthetic rubber obtained in an analysis method using a general GPC poorly corresponds to physical properties for processing such as Mooney viscosity, there has been fear that an intrinsic molecular weight distribution is not obtained in this method. This is because, among solvent- soluble components which constitute natural rubber or a diene- based synthetic rubber, an ultra- high molecular weight component is excluded by a filter (pore size: 0.45 $\mu$m) during filter filtration which is a pretreatment of GPC and the component is not included in the molecular weight distribution obtained by GPC. Further, as an experimental fact, when a THF solution of natural rubber is passed through a filter having a pore size of 0.45 $\mu$m by means of pressurized filtration or the like, the filter is soon clogged to increase the pressure, whereby it takes very long time for filtration, which has been problematic. This is because natural rubber contains a cross- linked gel component which is insoluble in THF, and an ultra- high molecular weight component dissolved in THF which does not pass through the filter of 0.45 $\mu$m. On the other hand,  in GPC, since it is possible that the component which does not pass through the filter of 0.45 $\mu$m does not pass through a column which has at the entrance and the exit a porous perforated filter such as a mesh or a frit and with which a fine filler is filled, a pretreatment for filter filtration is indispensable, and therefore, an ultra- high molecular weight component is excluded in a conventional method in which such a filter filtration is indispensable. Accordingly, an analysis method of natural rubber and a diene- based synthetic rubber in which an ultra- high molecular weight component which is soluble in an organic solvent and which does not pass through a filter of 0.45 $\mu$m can be analyzed has been demanded.
Accordingly, an object of the present invention is to solve the above- mentioned problems and to provide a rubber analysis method in which an analysis of natural rubber and a diene- based synthetic rubber including an ultra-high molecular weight component which could not be analyzed in a conventional method.

Means for Solving the Problems

The present inventor intensively studied to find that, by using a method in which a solution in which natural rubber and/or diene-based synthetic rubber is/are dissolved in an organic solvent is subjected to an ultracentrifugation in a centrifugal acceleration range of from 10,000 G to 1,000,000 G to separate a soluble component and an insoluble component in the solution, only a soluble component containing an ultra-high molecular weight component can be picked out to be analyzed, thereby completing the present invention.

Specifically, the rubber analysis method of the present invention is characterized by comprising: a dissolution process in which natural rubber and/or diene- based synthetic rubber is/are dissolved in an organic solvent; and a separation process in which a solution in which the natural rubber and/or diene- based synthetic rubber is/are dissolved is subjected to a centrifugation at a centrifugal acceleration of from 10, 000 G to 1, 000, 000 G, to separate a soluble component and an insoluble component in the solution.

In the present invention, the separated soluble component in the solution can be analyzed by an FFF device, more particularly, can be analyzed by an FFF device to which a MALS detector is connected for measuring the molecular weight and radius of gyration (hereinafter, also referred to as "FFF-MALS"). The FFF device herein refers to a device in which a sample solution is passed through a gap of from 100 $\mu$m to 500 $\mu$m (channel) and a field (field) is applied when the sample solution is passed through the channel, and therefore a molecular weight separation can be performed. For the field application method, a centrifugal force, a temperature difference or an electric field is employed. An asymmetrical flow FFF device in which a wall of the gap on one side is set to a semipermeable membrane, and in which a solvent is sucked with a syringe pump from the wall is suitable.

In the present invention, as the FFF device, those in which one of or both a MALS detector and single-angle light scattering instrument is/are connected to a viscosity detector are used, to thereby analyze the molecular weight and branching index of the separated soluble component in the solution. Further, in the present invention, the centrifugation is preferably performed by using an ultracentrifuge which can make a centrifuging portion in a vacuum state, and is also preferably performed by using a metal centrifuge tube.

In the separation process, the centrifugal acceleration of the centrifugation is suitably, 30,000 to 500,000 G, more suitably, 100,000 to 200,000 G. Further, as the organic solvent, at least one selected from the group consisting of tetrahydrofuran, chloroform, toluene and cyclohexane can be suitably used, and more suitably, tetrahydrofuran is used.

Effect of the Invention

In the present invention, by employing the above constitution, a rubber analysis method can be achieved in which natural rubber and a diene-based synthetic rubber including an ultra-high molecular weight component which could not be analyzed by a conventional method can be analyzed.

Brief Description of the Drawings

Fig. 1 is a graph showing the elution curve (A) of natural rubber obtained by FFF-MALS, and molecular weight (B) at each point on the elution curve.

Fig. 2 is a graph showing the differential molecular weight distribution curve (A) of natural rubber obtained by FFF-MALS and the differential molecular weight distribution curve (B) of natural rubber obtained by GPC-MALS.

Fig. 3 is a graph showing Log-Log plot of the radius of gyration and the molecular weight of natural rubber obtained by FFF-MALS, and (A) is a line of natural rubber, and (B) is a line obtained from a linear standard reference material.

Fig. 4 is a graph showing the distribution of the number of branching points /molecule of natural rubber measured by FFF-MALS.

Fig. 5 is a graph showing the elution curve (A) of natural rubber obtained by GPC-MALS, and molecular weight (B) at each point on the elution curve.

Fig. 6 is a graph showing Log-Log plot of the radius of gyration and the molecular weight of natural rubber obtained by GPC-MALS, and (A) is a line of natural rubber, and (B) is a line obtained from a linear standard reference material.

Fig. 7 is an explanatory diagram showing Debye plot.

Modes for Carrying out the Invention

Embodiments of the present invention will now be described in detail with reference to the Drawings.

In the rubber analysis method of the present invention, at first, natural rubber and/or a diene-based synthetic rubber to be analyzed is/are dissolved in an organic solvent (dissolution process), and then, the dissolved solution of natural rubber and/or diene-based synthetic rubber is/are subjected to a centrifugation at a centrifugal acceleration of from 10,000 G to 1,000,000 G to separate a soluble component and an insoluble component in the solution (separation process). Although centrifugation is a common separation technique, in the present invention, by using a method of a so-called ultracentrifugation at a centrifugal acceleration of from 10,000 G to 1,000,000 G, only soluble component including an ultra-high molecular weight component in a solution of natural rubber and/or a diene-based synthetic rubber can be picked out, and analysis of natural rubber and/or diene-based synthetic rubber including an ultra-high molecular weight component which could not conventionally analyzed can be performed.

In the present invention, natural rubber refers to a rubber which comprises, as a main backbone, polyisoprene obtained from a sap produced by plant species such as *Hevea brasiliensis.* The diene-based synthetic rubber refers to a synthetic rubber including a polymer obtained by polymerizing monomers composed of diene hydrocarbons such as butadiene and/or isoprene as a main backbone, and examples thereof include synthetic isoprene rubber (IR), butadiene rubber (BR) and styrene butadiene rubber (SBR). The organic solvent used for dissolving natural rubber and/or a diene-based synthetic rubber is not particularly restricted, and any solvent may be used as long as it can dissolve natural rubber and/or a diene-based synthetic rubber. Specifically, at least one selected from the group consisting of tetrahydrofuran (THF), chloroform, toluene and cyclohexane may be used alone or in combination. Among those, suitably, THF having an excellent solubility is used. The amount of natural rubber and/or diene-based synthetic rubber added to the organic solvent (amount of dissolution) may be, for example, from 0.001 to 1 mass% as the concentration of natural rubber and/or diene-based synthetic rubber in the solution. When the addition amount is too large, natural rubber and/or diene-based synthetic rubber is not sufficiently dissolved; on the other hand, when the addition amount is too small, the rubber(s) can not be detected by an RI (differential refractive index) detector used as a concentration detector.

In the above-mentioned dissolution process, for example, into the organic solvent, natural rubber and/or a diene-based synthetic rubber is/are put and then the solution is left to stand still for 12 hours to completely dissolve natural rubber and/or a diene-based synthetic rubber. In this case, in order to suppress deterioration of natural rubber and/or a diene-based synthetic rubber, antioxidants such as BHT (dibutylhydroxytoluene) may be added thereto.

In the separation process, a solution in which natural rubber and/or a diene-based synthetic rubber is/are dissolved is subjected to centrifugation at a centrifugal acceleration of from 10,000 G to 1,000,000 G, suitably from 30,000 to 500,000 G and more suitably from 100,000 to 200,000 G for from 10 minutes to 300 minutes. By this, a soluble component and an insoluble component in the solution can be separated. When the centrifugal acceleration is less than 10,000 G, the separation becomes insufficient. On the other hand, the container does not have sufficient durability for a centrifugal acceleration of larger than 1,000,000 G, and thus, the use of a centrifugal acceleration larger than 1,000,000 G is not practical. In the present invention, as the above container for centrifugation, a metal container which has a solvent resistance for THF is preferred, and further, a container made of stainless is preferably used in view of the durability. Here, in the present invention, the above centrifugation is preferably performed by making a centrifuging portion in a vacuum state and by using an ultracentrifuge in which a sample container can be rotated at an ultrahigh speed.

By collecting, from the solution of natural rubber and/or diene-based synthetic rubber which has been separated in the above-mentioned dissolution process and separation process, a supernatant liquid, soluble component containing an ultra-high molecular weight component is obtained. In the present invention, the analysis of the soluble component can be performed by a device in which an FFF device which is a molecular weight separation apparatus, a MALS detector or a single-angle light scattering detector such as a LALLS (Low angle laser light scattering) or RALLS (Right angle laser light scattering) which is a molecular weight and branch detector and a viscosity detector are connected. FFF is a technique in which ingredients in a solution can be fractionated by molecular weight using the difference of diffusion rates thereof, and does not need filter filtration as a pretreatment for a solution to be analyzed such as in a conventional GPC. In FFF, components in a solution are sequentially eluted from low molecular weight molecules having a large diffusion rate. Accordingly, by using an FFF device in place of a conventional GPC, soluble components including an ultra-high molecular weight component which has been conventionally excluded can be analyzed without performing filter filtration. As the FFF device, an asymmetrical flow FFF device is particularly preferably used.

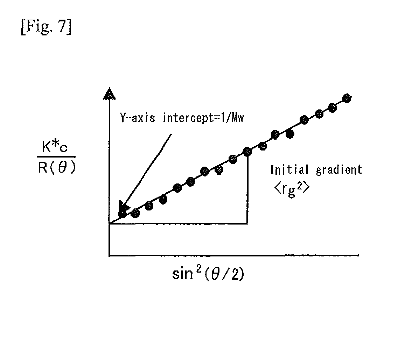

Here, the molecular weight distribution of the soluble component is obtained by measuring each molecular weight component separated by FFF using a MALS detector and using Debye plot (see Fig. 7). In the figure, $M_w$ represents the molecular weight, $K^*$ an optical parameter, c the concentration, $R(\theta)$ the Rayleigh ratio of the excessive scattering, and $r_g$ the radius of gyration. In this case, generally, the concentration of the soluble component is measured by using a differential refractive index (RI) detector. By this process, the weight-average molecular weight and the molecular weight distribution can be calculated. Further, by the gradient of the Debye plot, the radius of gyration ($r_g$) can be obtained, and the branching index g of each molecular weight component can be calculated. The branching index refers to a parameter representing the degree of branching represented by the following formula (a) :

$$g = \langle Rg_{branch}\rangle^2 / \langle Rg_{Linear}\rangle^2 \quad \cdots\cdots\cdots \ (a).$$

When the molecular structure of the sample is linear polymer, the branching index is 1. The larger the degree of branching, the lesser the branching index is than 1. Here, $\langle Rg_{branch}\rangle^2$ is the mean square of the radius of gyration of the sample, and $\langle Rg_{Linear}\rangle^2$ is the mean square of the radius of gyration of a linear standard reference material. Further, letting the number of branching points /molecule = B, the branching index g when the branching point has three functionality is represented by

$$g = [(1 + B/7)^{1/2} + 4B/9\pi]^{-1/2} \quad \cdots\cdots\cdots \ (b),$$

while the branching index g when the branching point has four functionalities is represented by

$$g = [(1 + B/6)^{1/2} + 4B/3\pi]^{-1/2}.$$

For each molecular weight component separated by FFF, the molecular weight distribution and weight-average molecular weight can be obtained also by the measurement using both single-angle light scattering instrument such as a RALLS device or a LALLS device and a viscosity detector, In this case, since the intrinsic viscosity is obtained by the viscosity detector, the branching index g is obtained by the intrinsic viscosity using the following formula:

$$g^b = [\eta]_{branch} / [\eta]_{Linear}.$$

Here, $[\eta]_{branch}$ represents the intrinsic viscosity of the sample, $[\eta]_{Linear}$ the intrinsic viscosity of the linear standard reference material, b a parameter determined by a branching structure such as star- type (b = 0.5), random-type (b = 1.5) . Specific examples of the calculation method of the branching index include (I) a method in which data of a practical linear standard reference material is used, (II) a method in which the conformation plot of a sample (Log-Log plot of the radius of gyration and the molecular weight) is drawn, and, assuming that the low molecular weight side of the sample is linear, the gradient and Y- axis intercept for a line of linear polymer are determined by extrapolation, or (III) a method of the combination of (I) and (II) in which data of the linear standard reference material is used only for the low molecular weight side and extrapolation is performed on the high molecular weight side.

On the other hand, by using a GPC-MALS method which is a combination of GPC and MALS, the branching index is theoretically obtained, but in the case of natural rubber, since it has many long chain branches, a function of separation according to molecular weight does not work well in GPC, and a low molecular weight linear polymer and a high molecular weight branched polymer elute in the same retention time due to an abnormal elution phenomenon, whereby the branching index can not be measured in a wide molecular weight range. For a natural rubber and diene-based synthetic rubber having many branches, the branching index thereof is a structural factor which is not obtained by a GPC-MALS analysis.

On the other hand, by drying and weighing a precipitated insoluble component corresponding to a cross-linked gel among the natural rubber and/or a diene-based synthetic rubber solution separated in the above-mentioned dissolution process and separation process, the gel fraction of natural rubber and/or a diene-based synthetic rubber can be evaluated.

Since by using the rubber analysis method of the present invention, the molecular weight distribution or structural factor such as a branch or gel fraction of natural rubber and/or diene-based synthetic rubber including an ultra-high molecular weight component which conventionally could not be analyzed can be evaluated, the relationship between physical properties for processing of the natural rubber and/or a diene-based synthetic rubber and the structure thereof is made clear, thereby contributing to development and quality control of natural rubber and/or a diene-based synthetic rubber.

Example

The present invention will now be explained in detail by way of Example and Comparative Example.

(Example)

Natural rubber sample (natural rubber manufactured by PT Bridgestone Sumatra Rubber Estate is used) was prepared, and the sample was added to THF such that the concentration of natural rubber in the solution was 0.4 mass%. The solution was left to stand still for 24 hours, and then, the solution was subjected to ultracentrifugation at a centrifugal acceleration of about 150,000 G for one hour by using a centrifuge tube made of stainless to separate a soluble component and an insoluble component in the solution. The weight of the insoluble component was measured after drying the precipitate to be 8.9 mass% based on the original natural rubber. The supernatant liquid corresponding to the separated soluble component in the solution was collected and diluted two-fold to be analyzed by FFF-MALS. As an FFF device, AF2000 manufactured by Postnova, as a MALS detector, Dawn Heleos II manufactured by Wyatt, and as an RI detector, PN3140 type manufactured by Postnova was used respectively. In this case, regarding the method of connecting the pipes for the device, FFF device -MALS detector -RI detector were connected in the order mentioned.

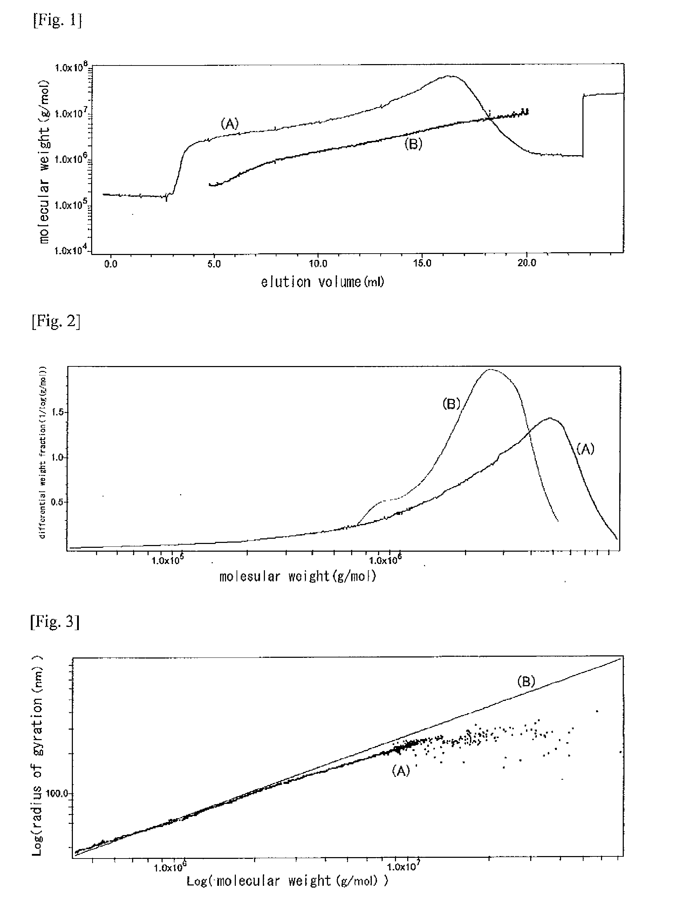

Fig. 1 is a graph showing the elution curve (A) of natural rubber obtained by FFF-MALS measurement, and molecular weight (B) at each point on the elution curve, by which a differential molecular weight distribution curve of natural rubber represented by (A) in Fig. 2 is obtained. Since in FFF-MALS measurement, the radius of gyration of each point on the elution curve is obtained, a Log-Log plot of radius of gyration and molecular weight (conformation plot) represented by (A) in Fig. 3 is obtained. Here, assuming that low molecular weight portion of natural rubber is a linear molecule, comparison with a line (B) corresponding to a linear polymer in which the gradient of the conformation plot of the low molecular weight portion of natural rubber is extrapolated to the high molecular weight side allows comparison of degree of branching. As depicted in Fig. 3, the higher the molecular weight, the lower the value of (A) than the line (B); therefore, the higher the molecular weight, the larger the degree of branching.

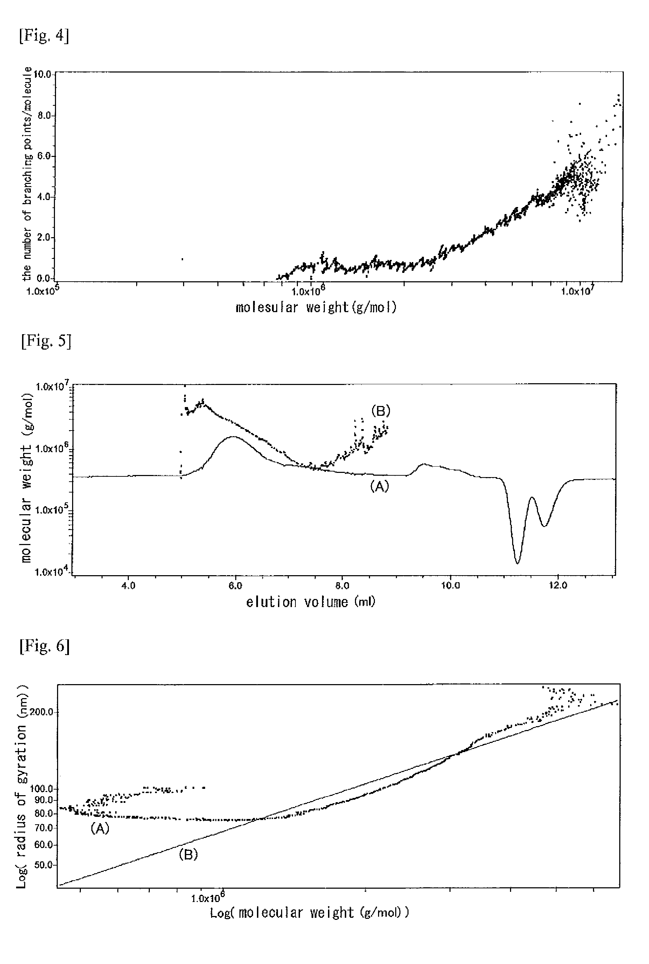

Further, based on the branching index g (formula (a) ) for each molecular weight, and assuming that the branching point has three functionalities, the distribution of the number of branching points /molecule as depicted in Fig. 4 is obtained by the formula (b) .

(Comparative Example)

The 0.4 mass% solution of natural rubber which has been left to stand still for 24 hours in Example was diluted four-fold, and then passed through a filter of 0.45 $\mu$m, and a GPC-MALS measurement was performed by using the filtrate. The same device as used in Example was used except that two GPC columns of TSK-GEL GMH(20) were used in place of channels needed for FFF separation.

Fig. 5 depicts the elution curve (A) of natural rubber obtained by GPC-MALS, and molecular weight (B) at each point on the elution curve. As depicted in (B) in Fig. 5, in the case of GPC-MALS, the molecular weight versus the elution volume represents not a straight line but a downward-convex curve. This is because, in the case of natural rubber, an abnormal elution phenomenon occurs in which a linear low molecule and a branched high molecule elute simultaneously. In GPC-MALS, an ultra-high molecular weight component dissolved in THF is not measured since filter filtration of 0.45 $\mu$m is performed as a pretreatment, and therefore, Fig. 2(B) depicts a differential molecular weight distribution curve which does not have a portion of the higher molecular weight compared with the result of GPC-MALS (Fig. 2(A)), which is different from the true molecular weight distribution.

In a GPC-MALS, as shown in Fig. 6, a Log-Log plot (A) of the radius of gyration and the molecular weight of natural rubber is usually above the line (B) obtained from a linear standard reference material, by which the number of branching points can not be calculated.

**Claims**

1. A rubber analysis method comprising: a dissolution process in which natural rubber and/or diene-based synthetic rubber is/are dissolved in an organic solvent; and a separation process in which a solution in which the natural rubber and/or diene-based synthetic rubber is/are dissolved is subjected to a centrifugation at a centrifugal acceleration of from 10,000 G to 1,000,000 G, to separate a soluble component and an insoluble component in the solution.

2. The rubber analysis method according to claim 1, wherein the separated soluble component in the solution is analyzed by a field flow fractionation device.

**3.** The rubber analysis method according to claim 2, wherein, as the field flow fractionation device, those to which a multi-angle light scattering detector is connected is used.

**4.** The rubber analysis method according to claim 2, wherein, as the field flow fractionation device, an asymmetrical flow field flow fractionation device is used.

**5.** The rubber analysis method according to claim 1, wherein, in the separation process, the centrifugal acceleration of centrifugation is from 30,000 to 500,000 G.

**6.** The rubber analysis method according to claim 5, wherein, in the separation process, the centrifugal acceleration of centrifugation is from 100,000 to 200,000 G.

**7.** The rubber analysis method according to claim 1, wherein the organic solvent is at least one selected from the group consisting of tetrahydrofuran, chloroform, toluene and cyclohexane.

**8.** The rubber analysis method according to claim 7, wherein the organic solvent is tetrahydrofuran.

**9.** The rubber analysis method according to claim 2, wherein, as the field flow fractionation device, those to which a multi-angle light scattering detector is connected  is used, and the molecular weight and the radius of gyration of the separated soluble component in the solution are analyzed.

**10.** The rubber analysis method according to claim 2, wherein, as the field flow fractionation device, those in which one of or both a multi-angle light scattering detector and single-angle light scattering instrument is/are connected to a viscosity detector are used, to thereby analyze the molecular weight and branching index of the separated soluble component in the solution.

**11.** The rubber analysis method according to claim 1, wherein the centrifugation is performed by using an ultracentrifuge which can make a centrifuging portion in a vacuum state.

**12.** The rubber analysis method according to claim 1, wherein the centrifugation is performed by using a metal centrifuge tube.

[Fig. 1]

[Fig. 2]

[Fig. 3]

[Fig. 4]

[Fig. 5]

[Fig. 6]

[Fig. 7]

<table>
<tr><td colspan="2"><strong>INTERNATIONAL SEARCH REPORT</strong></td><td>International application No.</td></tr>
<tr><td colspan="2"></td><td>PCT/JP2011/078317</td></tr>
</table>

A. CLASSIFICATION OF SUBJECT MATTER
*G01N15/06*(2006.01)i, *G01N1/10*(2006.01)i, *G01N21/49*(2006.01)i, *G01N33/44*
(2006.01)i

According to International Patent Classification (IPC) or to both national classification and IPC

B. FIELDS SEARCHED

Minimum documentation searched (classification system followed by classification symbols)
G01N15/06, G01N1/10, G01N21/49, G01N33/44

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | FULTON W S, GROVES S A, Determination of the Molecular Architecture of Synthetic and Natural Rubber by the Use of Thermal Field-flow Fractionation and Multi-angle Laser Light Scattering., J Nat Rubber Res, 1997, Vol.12 No.3, 154-165 | 1-12 |
| Y | LEE S, MOLNA´ R A, Determination of Molecular Weight and Gel Content of Natural Rubber Using Thermal Field-Flow Fractionation., Macromolecules, 1995.08.28, Vol.28 No.18, 6354-6356 | 1-12 |

☒ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 20 January, 2012 (20.01.12) | 31 January, 2012 (31.01.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2011/078317

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2000-319316 A   (Denki Kagaku Kogyo Kabushiki Kaisha), 21 November 2000 (21.11.2000), paragraphs [0006], [0007], [0014], [0018] (Family: none) | 1-12 |
| Y | JP 4-170409 A   (Ube Cycon, Ltd.), 18 June 1992 (18.06.1992), page 6, lower left column, line 2 to upper right column, line 2 (Family: none) | 1-12 |
| A | JP 50-123397 A   (Showa Denko Kabushiki Kaisha), 27 September 1975 (27.09.1975), page 1, lower right column, lines 8 to 13; page 2, upper left column, line 20 to upper right column, line 7 (Family: none) | 1-12 |
| Y | Karl-Gustav Wahlund and J. Calvin Giddings, Properties of an Asymmetrical Flow Field-Flow Fractionation Channel Having One Permeable Wall, Anal. Chem., 1987, Vol.59, 1332-1339 | 4 |
| Y | JP 11-230962 A   (Osaka Gas Co., Ltd.), 27 August 1999 (27.08.1999), paragraph [0030] (Family: none) | 10 |
| Y | JP 2010-058089 A   (Hitachi Koki Co., Ltd.), 18 March 2010 (18.03.2010), paragraph [0002] (Family: none) | 11 |
| Y | JP 61-057310 A   (Kuraray Co., Ltd.), 24 March 1986 (24.03.1986), claims & US 4699742 A              & EP 177727 A2 & DE 3566880 D | 12 |

Form PCT/ISA/210 (continuation of second sheet) (July 2009)

**EP 2 650 669 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Journal of Natural Rubber Research,* 1997, vol. 12 (3), 154-165
- *Macromolecules,* 1995, vol. 28, 6354-6356
- *Bull. Korean Chem. Soc.,* 2000, vol. 21 (1), 69-74